# EUROPEAN PATENT APPLICATION

(11) **EP 4 640 214 A1**
(43) Date of publication of application: **29.10.2025**
(21) Application number: 25166537.8
(22) Date of filing: 27.03.2025
(51) Int. Cl.: A61K 9/20, A61K 31/675

(54) **A TABLET COMPRISING TENOFOVIR ALAFENAMIDE MONOFUMARATE**

(30) Priority: 28.03.2024 TR 202403829
(71) Applicant: Sanovel Ilac Sanayi Ve Ticaret Anonim Sirketi, Istanbul (TR)
(72) Inventor: SUNEL, FATÍH, Istanbul (TR); ATAK, FADIME BILGEHAN, Istanbul (TR); IPEK, CELALEDDIN, Istanbul (TR); GENCAL, AYNUR, Istanbul (TR); ATAMAN, SEVAL, Istanbul (TR); KAPTAN, AYCA, Istanbul (TR); DERELI, SELIN, Istanbul (TR)
(74) Representative: Genç Ilhan, Oznur

(57) **Abstract**

The present invention relates to a tablet composition comprising tenofovir alafenamide monofumarate and at least one pharmaceutically acceptable excipient, wherein tenofovir alafenamide monofumarate has a d (0.9) particle size between 85 µm and 20 µm. The present invention also relates to a simple, rapid, cost effective, time-saving and a solvent-free process of preparing the composition.

## Description

### Field of Invention

The present invention relates to a tablet composition comprising tenofovir alafenamide monofumarate and at least one pharmaceutically acceptable excipient, wherein tenofovir alafenamide monofumarate has a d (0.9) particle size between 85 µm and 20 µm. The present invention also relates to a simple, rapid, cost effective, time-saving and a solvent-free process of preparing the composition.

### The Background of The Invention

Tenofovir is an antiviral medication commonly used to treat long-term hepatitis B virus infection, which can cause inflammation and damage to the liver. Tenofovir reduces the amount of hepatitis B virus in your body to limit damage to your liver. It is a tablet which should be taken by mouth.

Tenofovir is a nucleotide analogue reverse transcriptase inhibitor. The chemical name of tenofovir is ({[(2R)-1-(6-amino-9H-purin-9-yl)propan-2-yl]oxy}methyl)phosphonic acid and has the structure shown in the following Formula I;

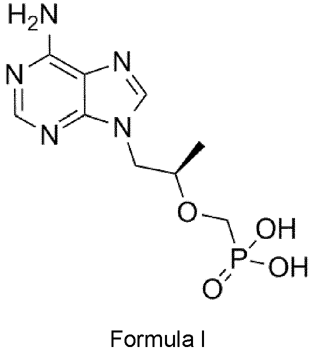

Tenofovir alafenamide monofumarate can be represented by the following chemical structure according to Formula II:

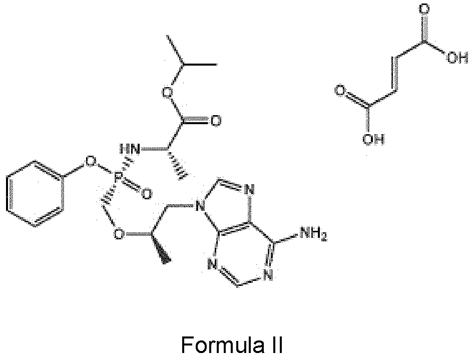

WO 02/008241 A2 discloses tenofovir alafenamide and a tenofovir alafenamide monofumarate form as well as processes for their preparations.

There are products on the market containing tenofovir or its salts. The prior art also contains many examples of patents relating to tenofovir or its salts.

Thus, there is still a need for a tablet composition comprising tenofovir alafenamide monofumarate, wherein the stability of the active pharmaceutical ingredient and content uniformity can be ensured. In particular, a pharmaceutical composition in which the degradation of the active pharmaceutical ingredient is prevented or at least significantly reduced even under harsh conditions is provided.

### Detailed Description of The Invention

The main object of the present invention is to provide a tablet composition comprising tenofovir alafenamide monofumarate with high content uniformity and improved powder flow properties which overcomes the above-described problems in prior art and have additive advantages over them.

Another object of the present invention is to obtain a tablet composition comprising tenofovir alafenamide monofumarate having the desired drug release profil and high stability.

Another object of the present invention is to provide a process for a tablet composition comprising tenofovir alafenamide monofumarate with high stability.

Tenofovir alafenamide monofumarate used in the formulation has a high proportion by weight. Tenofovir alafenamide monofumarate should be blended effectively in the formulation and be homogeneous in the whole tablet and content uniformity should be ensured. In our studies for this, we have seen that homogeneity and desired drug release profil were achieved by using tenofovir alafenamide monofumarate in the particle size specified in claim 1. We have also seen that we can overcome the poor compressibility of tenofovir alafenamide monofumarate in this way.

As used here in, 'particle size' means the cumulative volume size distribution as tested by any conventionally accepted method such as the laser diffraction method (i.e. Malvern analysis). The term d (0.9) means the size at which %90 by volume of the particles are finer, the term d (0.5) means the size at which %50 by volume of the particles are finer, the term d (0.1) means the size at which %10 by volume of the particles are finer.

According to one embodiment, a tablet composition comprises tenofovir alafenamide monofumarate and at least one pharmaceutically acceptable excipient, wherein tenofovir alafenamide monofumarate has a d (0.9) particle size between 85 µm and 20 µm.

According to one embodiment, tenofovir alafenamide monofumarate has a d (0.9) particle size between 80 µm and 24 µm or between 75 µm and 28 µm or between 70 µm and 35 µm.

According to one embodiment of the invention, the amount of tenofovir alafenamide monofumarate is between 5.0% to 20.0%, preferably 9.0% to 18.0%, preferably 15.0% to 17.0% by weight of the total core tablet.

According to one embodiment of the invention, a tablet composition comprises at least one pharmaceutically acceptable excipient is selected from the group comprising fillers, disintegrants, lubricants, glidants or mixtures.

Factors determining stability of drug substance include factors such as light/moisture/temperature sensivity, pH and other excipients and process design. In this invention, low moisture content is important because tenofovir alafenamide monofumarate degrade at a high rate when exposed to humidity. Therefore, the loss on drying limit of the filler, which is the most commonly used pharmaceutically acceptable excipient in the composition, is very important.

Suitable fillers are selected from a group comprising microcrystalline cellulose, lactose monohydrate, lactose, mannitol, spray-dried mannitol, starch, dextrose, sucrose, fructose, maltose, sorbitol, inorganic salts, calcium salts, polysaccharides, dicalcium phosphate, sodium chloride, dextrates, lactitol, maltodextrin, sucrose-maltodextrin mixture, trehalose, sodium carbonate, sodium bicarbonate, calcium carbonate, dextrose, maltitol or mixtures thereof.

According to one embodiment of the invention, the filler is microcrystalline cellulose or lactose monohydrate or mixtures thereof.

According to one embodiment of the invention, the loss on drying limit of the filler is <1.5%. Halogen moisture analyzer is measured by thermogravimetric method, the moisture content is calculated by the weight loss caused by heating the sample.

According to one embodiment of the invention, the amount of the fillers is 40.0% to 95.0%, preferably 60.0% to 85.0%, preferably 70.0% to 80.0% by weight of the total core tablet.

Suitable disintegrants are selected from a group comprising croscarmellose sodium, sodium lauryl sulphate, povidone, pregelatinized starch, sodium carboxymethyl cellulose, calcium carboxymethyl cellulose, carboxymethyl cellulose, docusate sodium, low substituted hydroxypropyl cellulose, polyacryline potassium, sodium alginate, corn starch, sodium starch glycolate, ion-exchange resins, magnesium aluminium silica, sodium dodecyl sulphate, poloxamer, sodium glycine carbonate or mixtures thereof.

According to one embodiment of the invention, the disintegrant is croscarmellose sodium.

According to one embodiment of the invention, the amount of the disintegrants is 1.0% to 15.0%, preferably 4.0% to 9.0% by weight of the total core tablet.

Suitable lubricants are selected from the group comprising magnesium stearate, calcium stearate, zinc stearate, boric acid, hydrogenated vegetable oil, sodium chlorate, magnesium lauryl sulfate, sodium oleate, sodium acetate, sodium benzoate, polyethylene glycol or mixtures thereof.

According to one embodiment of the invention, the lubricant is magnesium stearate.

According to one embodiment of the invention, the amount of the lubricants is 0.5% to 5.0%, preferably 0.75% to 2.5% by weight of the total core tablet.

Suitable glidants are selected from group comprising colloidal silicon dioxide, water soluble excipient, hydrophilic polymers, silicon dioxide or mixtures thereof.

According to one embodiment of the present invention, the glidant is colloidal silicon dioxide.

According to one embodiment of the invention, the amount of the glidants is 0.5% to 4.0%, preferably 0.75% to 2.0% by weight of the total core tablet.

According to one embodiment, the tablet composition comprises;
- Tenofovir Alafenamide Monofumarate
- Lactose monohdrate
- Microcrytalline cellulose
- Croscarmellose sodium
- Magnesium stearate

According to one embodiment, the tablet composition comprises;
- Tenofovir Alafenamide Monofumarate
- Lactose monohdrate
- Microcrytalline cellulose
- Colloidal silicon dioxide
- Croscarmellose sodium
- Magnesium stearate

According to one embodiment, the tablet composition comprises;
- 5.0 to 20.0% by weight tenofovir alafenamide monofumarate
- 25.0 to 55.0% by weight lactose monohdrate
- 15.0 to 40.0% by weight microcrytalline cellulose
- 1.0 to 15.0% by weight croscarmellose sodium
- 0.5 to 5.0% by weight magnesium stearate.

The active ingredient tenofovir alafenamide monofumarate in the present invention is extremely easy to hydrolyze. Therefore, it is very important to choose excipients and not to use solvents.

In addition, a solvent-free process (dry granulation or direct compression or roller compaction) is used for tenofovir alafenamide monofumarate and excipients to improve the flowability, and then perform the subsequent tableting process. The selected excipients and the process in the composition of the present invention are particularly suitable for powder's their excellent compressibility and flowability.

According to one embodiment, a packaging comprising tenofovir alafenamide monofumarate further comprises aluminium foil with desiccant used to provide moisture protection.

The following examples illustrate the composition according to the invention and the advantages of this formulation. However, they in no way represent a limitation of the present invention but simply illustrate the invention.

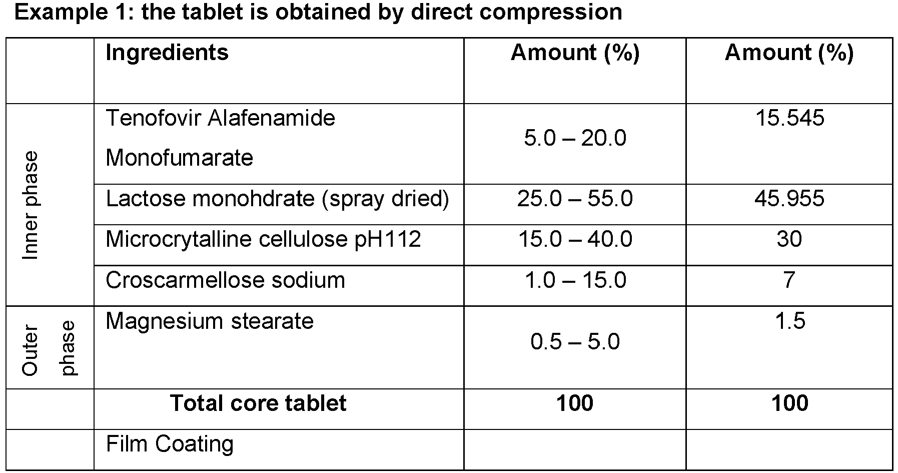

Process for example 1;
a) Mixing Tenofovir alafenamide monofumarate, lactose monohydrate, microcrystalline cellulose and croscarmellose sodium,
b) Adding magnesium stearate and then mixing,
c) Compressing the mixture into tablet form,
d) Coating the tablets.

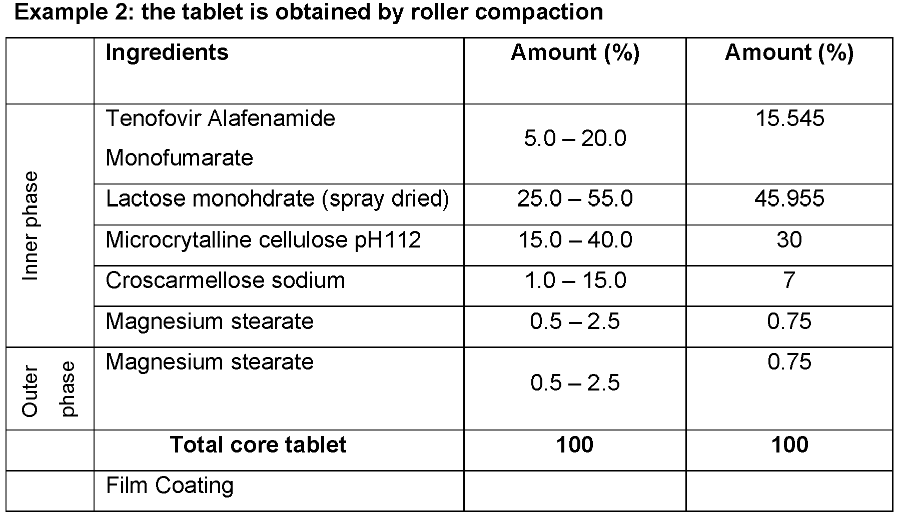

Process for example 2;
a) Mixing Tenofovir alafenamide monofumarate, lactose monohydrate, microcrystalline cellulose and croscarmellose sodium and magnesium stearate,
b) Taking this mixture to the roller compactor and then milling,
c) Adding magnesium stearate to the mixtures (b) and then mixing,
d) Compressing the mixture into tablet form,
e) Coating the tablets.

### Example 3: the tablet is obtained by direct compression

| **Ingredients** | | **Amount (%)** | **Amount (%)** |
|---|---|---|---|
| Tenofovir Alafenamide Monofumarate | | 5.0 - 20.0 | 15.545 |
| Lactose monohdrate (spray dried) | | 25.0 - 55.0 | 44.955 |
| Microcrytalline cellulose pH112 | | 15.0 - 40.0 | 30 |
| Colloidal silicon dioxide | | 0.5-4.0 | 1 |
| Croscarmellose sodium | | 1.0 - 15.0 | 7 |
| Magnesium stearate | | 0.5-5.0 | 1.5 |
| | **Total core tablet** | **100** | **100** |
| Film Coating | | | |

Process for example 3;
a) Mixing Tenofovir alafenamide monofumarate, lactose monohydrate, microcrystalline cellulose, colloidal silicon dioxide and croscarmellose sodium,
b) Adding magnesium stearate and then mixing,
c) Compressing the mixture into tablet form,
d) Coating the tablets.

### Film coating

| |
|---|
| Polyvinyl Alcohol |
| Titanium dioxide |
| Talc |
| Macrogol |
| Coloring agent |

## Claims

1. A tablet composition comprising tenofovir alafenamide monofumarate and at least one pharmaceutically acceptable excipient, wherein tenofovir alafenamide monofumarate has a d (0.9) particle size between 85 µm and 20 µm.

2. The tablet composition according to claim 1, wherein tenofovir alafenamide monofumarate has a d (0.9) particle size between 80 µm and 24 µm or between 75 µm and 28 µm or between 70 µm and 35 µm.

3. The tablet composition according to claim 1, wherein the amount of tenofovir alafenamide monofumarate is between 5.0% to 20.0%, preferably 9.0% to 18.0%, preferably 15.0% to 17.0% by weight of the total core tablet.

4. The tablet composition according to claim 1, wherein at least one pharmaceutically acceptable excipient is selected from the group comprising fillers, disintegrants, lubricants, glidants or mixtures.

5. The tablet composition according to claim 4, wherein fillers are selected from a group comprising microcrystalline cellulose, lactose monohydrate, lactose, mannitol, spray-dried mannitol, starch, dextrose, sucrose, fructose, maltose, sorbitol, inorganic salts, calcium salts, polysaccharides, dicalcium phosphate, sodium chloride, dextrates, lactitol, maltodextrin, sucrose-maltodextrin mixture, trehalose, sodium carbonate, sodium bicarbonate, calcium carbonate, dextrose, maltitol or mixtures thereof.

6. The tablet composition according to claim 5, wherein the filler is microcrystalline cellulose or lactose monohydrate or mixtures thereof.

7. The tablet composition according to claim 5 or 6, wherein the loss on drying limit of the filler is <1.5%.

8. The tablet composition according to claim 4, wherein disintegrants are selected from a group comprising croscarmellose sodium, sodium lauryl sulphate, povidone, pregelatinized starch, sodium carboxymethyl cellulose, calcium carboxymethyl cellulose, carboxymethyl cellulose, docusate sodium, low substituted hydroxypropyl cellulose, polyacryline potassium, sodium alginate, corn starch, sodium starch glycolate, ion-exchange resins, magnesium aluminium silica, sodium dodecyl sulphate, poloxamer, sodium glycine carbonate or mixtures thereof.

9. The tablet composition according to claim 8, wherein the disintegrant is croscarmellose sodium.

10. The tablet composition according to claim 8 or 9, wherein the amount of the disintegrants is 1.0% to 15.0%, preferably 4.0% to 9.0% by weight of the total core tablet.

11. The tablet composition according to any preceding claim, wherein the composition comprising;
- Tenofovir Alafenamide Monofumarate
- Lactose monohdrate
- Microcrytalline cellulose
- Croscarmellose sodium
- Magnesium stearate

12. The tablet composition according to any preceding claim, wherein the composition comprising;
- Tenofovir Alafenamide Monofumarate
- Lactose monohdrate
- Microcrytalline cellulose
- Colloidal silicon dioxide
- Croscarmellose sodium
- Magnesium stearate

13. The tablet composition according to any preceding claim, wherein the composition comprising;
- 5.0 to 20.0% by weight tenofovir alafenamide monofumarate
- 25.0 to 55.0% by weight lactose monohdrate
- 15.0 to 40.0% by weight microcrytalline cellulose
- 1.0 to 15.0% by weight croscarmellose sodium
- 0.5 to 5.0% by weight magnesium stearate.
